# EUROPEAN PATENT APPLICATION

(11) **EP 4 566 572 A2**
(43) Date of publication of application: **11.06.2025**
(21) Application number: 25153109.1
(22) Date of filing: 01.02.2021
(51) Int. Cl.: A61F 2/24

(54) **ENHANCED FLEXIBILITY ANNULOPLASTY BANDS**

(30) Priority: 06.02.2020 US 202062971139 P
(62) Divisional of application: 21707582.9
(71) Applicant: Edwards Lifesciences Corporation, Irvine, CA 92614 (US)
(72) Inventor: Rodriguez, Rodolfo, Irvine, CA, 92614 (US); Conklin, Brian S., Irvine, CA, 92614 (US); Campbell, Louis A., Irvine, CA, 92614 (US)
(74) Representative: Eisenführ Speiser

(57) **Abstract**

An annuloplasty band having a differentiation in area moment of inertia, where the area moment of inertia in the out of plane direction is much less than the area moment of inertia in the plane of the annulus. This makes the band stiff enough to hold the annulus in the correct shape while being flexible enough out of plane to minimize the risk of suture dehiscence or breakage. One example is a C-shaped band with a core formed of nitinol and having a constant cross-section with a wider radial dimension than an axial dimension. The cross-section may be rectangular. The band is asymmetric across a minor axis with one end extending around the anterior side farther than the other. The free ends rise up from adjacent lateral sides, and a continuous posterior mid-section also rises upward.

## Description

### TECHNICAL FIELD

The present disclosure relates generally to annuloplasty bands, and in particular to a mitral annuloplasty band having enhanced out-of-plane flexibility.

### BACKGROUND

In vertebrate animals, the heart is a hollow muscular organ having four pumping chambers: the left and right atria and the left and right ventricles, each provided with its own one-way valve. The natural heart valves are identified as the aortic, mitral (or bicuspid), tricuspid and pulmonary, and are each mounted in an annulus comprising dense fibrous rings attached either directly or indirectly to the atrial and ventricular muscle fibers. Each annulus defines a flow orifice. The four valves ensure that blood does not flow in the wrong direction during the cardiac cycle; that is, to ensure that the blood does not back flow through the valve. Blood flows from the venous system and right atrium through the tricuspid valve to the right ventricle, then from the right ventricle through the pulmonary valve to the pulmonary artery and the lungs. Oxygenated blood then flows through the mitral valve from the left atrium to the left ventricle, and finally from the left ventricle through the aortic valve to the aorta/arterial system.

The mitral and tricuspid valves are defined by fibrous rings of collagen, each called an annulus, which forms a part of the fibrous skeleton of the heart. The annulus provides peripheral attachments for the two cusps or leaflets of the mitral valve (called the anterior and posterior cusps) and the three cusps or leaflets of the tricuspid valve. The native valve leaflets flex outward when the valve opens and their free edges come together or coapt in closure.

The free edges of the mitral leaflets connect to chordae tendineae from more than one papillary muscle. Mitral valve malfunction can result from the chordae tendineae (the chords) becoming stretched, and in some cases tearing. Also, a normally structured valve may not function properly because of an enlargement of or shape change in the valve annulus. This condition is referred to as a dilation of the annulus and generally results from heart muscle failure. In addition, the valve may be defective at birth or because of an acquired disease. From a number of etiologies, mitral valve dysfunction can occur when the leaflets do not coapt at peak contraction pressures. As a result, an undesired back flow of blood from the left ventricle into the left atrium can occur.

Various surgical techniques may be used to repair a diseased or damaged valve. A commonly used repair technique effective in treating incompetence is annuloplasty, which often involves reshaping or remodeling the annulus by attaching a prosthetic annuloplasty repair segment or ring thereto. For instance, the goal of a posterior mitral annulus repair is to bring the posterior mitral leaflet forward toward to the anterior leaflet to better allow coaptation. The annuloplasty ring is designed to support the functional changes that occur during the cardiac cycle: maintaining coaptation and valve integrity to prevent reverse flow while permitting good hemodynamics during forward flow.

Annuloplasty rings may be stiff, flexible or semi-rigid, and a "remodeling" annuloplasty ring typically has an inner core that resists conforming to the native annulus shape and instead forces the annulus to conform to it. Remodeling annuloplasty bands or rings are "generally rigid" or "semi-rigid" in that they will resist distortion when subjected to the stress imparted thereon by the mitral valve annulus of an operating human heart. In this sense, "distortion" means substantial permanent deformation from a predetermined or manufactured shape (e.g., the band or ring will tend to return to its preset shape in use). A typical remodeling annuloplasty ring comprises an inner substrate or core of a metal such as a rod or multiple bands of stainless steel or titanium covered with a biocompatible fabric or cloth and perhaps silicone to allow the ring to be sutured to the fibrous annulus tissue. Annuloplasty rings have been associated with a 10% to 15% ring dehiscence (suture pullout) incidence at 10 years, thus requiring a reoperation. Some examples disclosed herein reduce this complication.

Annuloplasty rings may have a variety of shapes in plan view, including closed or continuous oval, circular, D-shaped, or kidney-shaped, and open or discontinuous C-shaped, sometimes referred to as a band. Examples are seen in U.S. Patent Nos. 5,041,130, 5,104,407, 5,201,880, 5,258,021, 5,607,471 and, 6,187,040. Most rigid and semi-rigid annular rings for the mitral valve have a kidney-like or D shape, with a curved posterior segment co-extensive with the posterior valve leaflet, and a somewhat straighter anterior segment co-extensive with the anterior valve leaflet.

One popular annuloplasty ring is the partially flexible Carpentier-Edwards Physio^{®} ring available from Edwards Lifesciences of Irvine, CA. The Physio^{®} ring is a closed "semi-rigid" ring because it offers selective flexibility at the posterior section while preserving the remodeling effect through a rigid anterior section. The newer Physio II^{®} ring from Edwards Lifesciences also features up and down curves to better fit the nonplanar contours of the mitral annulus. Various other rings have posterior bows, *e.g.,* U.S. Patent Nos. 6,805,710 and 6,858,039, 7,959,673, or other three-dimensional configurations.

More recently, U.S. Patent No. 10,314,707 to Adams discloses an open or C-shaped annuloplasty band shaped and sized to avoid the adjacent aortic valve structure and better protect against dehiscence along the muscular mitral annulus. The annuloplasty band has an inner core of various metals with a radial cross-section that gradually reduces in magnitude toward free ends of the core to supply greater flexibility at those locations.

Despite numerous designs presently available or proposed in the past, there is a need for an annuloplasty ring that both remodels the mitral annulus and reduces the chance of suture pull-out or dehiscence.

### SUMMARY

Examples of the present disclosure provide an annuloplasty band shaped and sized so as to have a differentiation in area moment of inertia, where the area moment of inertia in the out of plane direction is much less than the area moment of inertia in the plane of the annulus. This makes the band stiff enough to hold the annulus in the correct shape while being flexible enough out of plane to minimize the risk of suture dehiscence or breakage. One example is a C-shaped band with a core formed of nitinol and having a constant cross-section with a wider radial dimension than an axial dimension. The cross-section may be rectangular. The band is asymmetric across a minor axis with one end extending around the anterior side farther than the other. The free ends rise up from adjacent lateral sides, and a continuous posterior mid-section also rises upward.

The present application discloses a number of annuloplasty bands adapted for implant against a mitral valve annulus, the mitral valve annulus having a posterior aspect to which a posterior leaflet attaches and an anterior aspect to which an anterior leaflet attaches. Per convention, the mitral valve annulus generally defines a D- or kidney-shape looking at an inflow side thereof with the anterior aspect being straighter than the more rounded posterior aspect. A minor axis intersects and extends across the mitral valve annulus between mid-points on the anterior and posterior aspects, the minor axis being shorter than a major axis perpendicular thereto intersecting and extending across the mitral valve annulus, wherein an intersection of the minor and major axes defines a reference plane.

One exemplary annuloplasty band has an inner core formed of nitinol and covered by a suture-permeable interface. The inner core has a shape such that when the ring is implanted around the mitral annulus the core has a continuous posterior mid-section that extends around on each side past the major axis in first and second segments terminating in first and second free ends, respectively, the first and second segments being of different length. The core also has a constant cross-section throughout which is stiffer in bending within the reference plane than bending out of the reference plane.

A second exemplary annuloplasty band has an inner core formed of nitinol and covered by a suture-permeable interface. The inner core has a shape such that when the ring is implanted around the mitral annulus the core has a continuous posterior mid-section that extends around on each side past the major axis in first and second segments terminating in first and second free ends, respectively. The core has a constant rectangular cross-section throughout which is oriented to have a width generally parallel to the plane defined by the intersection of the major and minor axes, and a height which is perpendicular thereto, wherein the width is larger than the height.

A still further annuloplasty band adapted for implant against an atrioventricular valve annulus, comprises a one-piece inner core formed of nitinol and covered by a suture-permeable interface. The inner core has a shape such that when the ring is implanted around the valve annulus the core has a continuous posterior mid-section that extends around on each side and terminates in first and second free ends. The core further has a constant rectangular cross-section throughout which is oriented to have a width generally parallel to a plane which the ring primarily defines, and a height which is perpendicular thereto, and the width is larger than the height. The inner core has an in-plane stiffness of at least about 0.5 N/mm.

In any of the annuloplasty bands disclosed herein, the inner core has an in-plane stiffness of between about 0.5 and about 1 N/mm. More particularly, the inner core has an in-plane stiffness of at least about 0.6 N/mm, at least about 0.7 N/mm, or at least about 0.8 N/mm.

In any of the annuloplasty bands disclosed herein, the inner core has an out-of-plane stiffness to in-plane stiffness ratio of greater than about 2.5%. More particularly, the inner core has an out-of-plane stiffness to in-plane stiffness ratio of greater than about 3%, or greater than about 2%. In one example, the inner core has an out-of-plane stiffness to in-plane stiffness ratio of no more than about 6%. In another example, the inner core has an out-of-plane stiffness to in-plane stiffness ratio of about 1%, or about 1.5%, or about 2%, or about 2.5%, or about 3%, or about 3.5%, or about 4%, or about 4.5%, or about 5%, or about 5.5%, or about 6%, or about 6.5%, or about 7%. In a still further example, the inner core has an out-of-plane stiffness to in-plane stiffness ratio of between about 1% and about 7%, or between about 2% and about 5.5%%, or between about 3.5% and about 5.5%, or between about 2% and about 5%, or between about 2.5% and about 5%, or between about 3% and about 6%, or between about 3% and about 5.5%, or between about 2.5% and about 5.5%, or between about 4% and about 6%, or between about 3.5% and about 5%, or between about 2.5% and about 5.5%.

In another aspect, a first method of forming any of the annuloplasty bands disclosed herein includes laser cutting a planar blank from a sheet of nitinol having an approximate peripheral length of the inner core, bending the planar blank to a 3D shape to form the core, and heat treating the core.

In the first method of forming, the steps of bending and heat-treating may be repeated multiple times to avoid placing undue strains/stresses on the blank during bending. The step of bending may comprise clamping the blank between two fixtures having opposed surfaces that define the 3D shape. Further, the step of heat-treating may be done while the blank is clamped between the two fixtures.

A second method of forming any of the annuloplasty bands disclosed herein includes cutting a rectangular-cross-sectioned wire to a length, bending the wire to a 3D shape to form the core, and heat treating the core.

The second method may further include repeating the steps of bending and heat-treating two or more times to avoid placing undue strains/stresses on the wire during bending steps between heat treating steps. In addition, the step of bending may comprise clamping the wire between two fixtures having opposed surfaces that define the 3D shape. Further, the step of heat-treating may be done while the wire is clamped between the two fixtures. The wire may be cut to a length having an approximate peripheral length of the inner core. Where the wire is cut to a length longer than an approximate peripheral length of the inner core, the free ends of the wire may be inserted into and held within an inner cavity in one of the fixtures.

A further understanding of the nature and advantages of the disclosure will become apparent by reference to the remaining portions of the specification and drawings.

### BRIEF DESCRIPTION OF THE DRAWINGS

Features and advantages of the present disclosure will become appreciated as the same become better understood with reference to the specification, claims, and appended drawings wherein:
Figure 1 is a superior or plan view of a healthy mitral valve, with the leaflets closed and coapting at peak contraction pressures during ventricular systole and indicating the primary anatomical landmarks as well as diagram lines indicating the circumferential reach of bands of the present application;
Figure 2 is a plan view of a mitral valve as in Figure 1 with an exemplary annuloplasty band of the present application shown implanted therearound;
Figures 3A-3C are plan and elevational views of an exemplary annuloplasty band;
Figures 4A and 4B are sectional views of the exemplary annuloplasty band taken along corresponding sections lines in Figure 3A;
Figures 5A and 5B are perspective views of an exemplary inner core for the annuloplasty band of Figures 3A-3C;
Figures 6A-6C are plan and elevational views of an exemplary inner core for the annuloplasty band of Figures 3A-3C;
Figure 7A is a sectional view of the inner core taken along a corresponding section line in Figure 6A;
Figures 8A and 8B are perspective views of an exemplary core forming fixture of the present application;
Figure 9 is an exploded view of the core forming fixture of Figures 8A and 8B showing two different blanks that may be used to form the core;
Figures 10A and 10B are perspective views of an alternative core forming fixture of the present application;
Figure 11A is an exploded view of the core forming fixture of Figure 10A and 10B showing a blank that may be used to form the core; and
Figure 11B is an assembled view of the core forming fixture of Figure 10A and 10B with the blank secured therein.

### DETAILED DESCRIPTION OF CERTAIN EXAMPLES

The present application discloses a mitral annuloplasty band that avoids the adjacent aortic valve structure and better protects against dehiscence along the muscular mitral annulus. The term "band" is used here since the implant is an open or discontinuous ring, although in some contexts such implants are also termed "rings". Indeed, the bands disclosed herein define shapes that circumscribe a majority of the mitral annulus, and thus trace most of a ring shape. A complete ring shape may be constructed, and indeed the shape of the bands may be defined, by imagining an extension of the shape between and connecting the free ends. For example, the preferred plan view shape of the disclosed bands is kidney or D-shaped so as to conform to the peripheral shape of the usual mitral annulus. Therefore "band" and "ring" are synonymous, the disclosed band or ring simply being discontinuous so as to have two free ends.

The right ventricle RV and left ventricle LV are separated from the right atrium RA and left atrium LA, respectively, by the tricuspid valve TV and mitral valve MV; *e.g.,* the atrioventricular valves. Though correction of the mitral annulus is the primary focus of the present application, it should be understood that certain characteristics of the annuloplasty bands described herein may equally be used to treat the tricuspid valve TV, and thus the claims should not be constrained to the mitral band unless expressly limited.

The term "axis" in reference to the illustrated annuloplasty bands, and other non-circular or non-planar bands, refers to a line generally through the centroid of the band or ring periphery when viewed in plan view. "Axial" or the direction of the "axis" can also be viewed as being parallel to the average direction of blood flow within the valve orifice and thus within the band when implanted therein. Stated another way, an implanted mitral band or ring orients about a central flow axis aligned along an average direction of blood flow through the mitral annulus from the left atrium to the left ventricle.

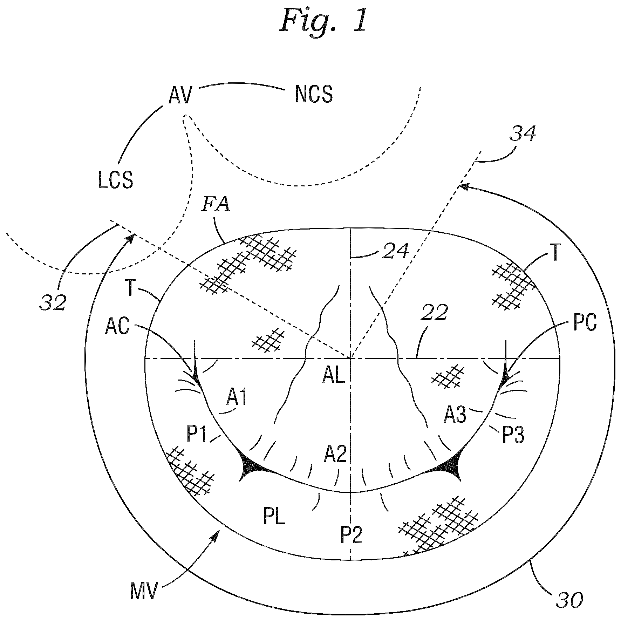

Figure 1 is a plan view of the mitral valve with posterior being down and anterior being up. In a healthy heart, the annulus of the mitral valve **MV** creates an anatomic shape and tension such that a posterior leaflet **PL** and an anterior leaflet **AL** coapt in the flow orifice, forming a tight junction, at peak contraction or systolic pressures, as seen in Figure 1. The mitral valve MV annulus has a posterior aspect to which the posterior leaflet **PL** attaches and an anterior aspect to which the anterior leaflet **AL** attaches. Where the leaflets meet at the opposing medial and lateral sides of the annulus are called the leaflet commissures: the anterior (or more accurately, the antero-medial) commissure **AC,** and the posterior (or postero-lateral) commissure **PC.** The posterior leaflet is divided into three scallops or cusps, sometimes identified as **P1, P2,** and **P3,** starting from the anterior commissure and continuing in a counterclockwise direction to the posterior commissure. The posterior scallops **P1, P2,** and **P3** circumscribe particular arcs around the periphery of the posterior aspect of the annulus, which may vary depending on a variety of factors, including actual measurement of the mitral valve posterior leaflet scallops, and surgeon preference. As a rule, however, a major axis 22 of the mitral annulus intersects both the first and third posterior scallops **P1** and **P3,** approximately at the commissures **AC, PC,** and a minor axis 24 intersects and generally bisects the middle posterior scallop **P2.** The anterior leaflet also features scallops or regions labeled **A1, A2,** and **A3** as indicated in Figure 1.

The mitral anterior leaflet **AL** attaches to the fibrous portion **FA** of the mitral annulus, which makes up about one-third of the total mitral annulus circumference. The muscular portion of the mitral annulus constitutes the remainder of the mitral annulus, and the posterior leaflet **PL** attaches thereto. The anterior fibrous annulus **FA,** the two ends of which are called the fibrous trigones T, forms part of the central fibrous skeleton of the heart. The anterior commissure **AC** and the posterior commissure **PC** are located just posterior to each fibrous trigone.

The fibrous mitral valve annulus **FA** is intimate with or adjacent to the aortic valve **AV,** in particular the left coronary sinus **LCS** and non-coronary sinus **NCS.** The central fibrous body is fairly resistant to elongation, and thus the great majority of mitral annulus dilation occurs in the posterior two-thirds of the annulus, or around the muscular mitral annulus.

In a preferred example, the mitral annuloplasty bands disclosed herein comprise discontinuous rings defining a kidney or D-shape with a substantially complete posterior segment centered about a minor axis of the band. Further, the annuloplasty band defines two anterior segments with free ends opposite each other and having differing lengths extending from the posterior segment. The different lengths of the two anterior segments of the band create an asymmetry which is imbalanced toward the posterior commissure.

To better define the contours of the asymmetric annuloplasty band disclosed herein, Figure 1 illustrates a circumferential span 30 around the mitral annulus, generally illustrating the length of the band. More particularly, the band length extends around in a counter-clockwise (CCW) direction between a radial angular line 32 located within the anterior fibrous annulus **FA** to a radial angular line 34 that is past the posterior commissure **PC** and also within the anterior fibrous annulus **FA.** The radial angular line 32 makes an angle from the vertical of about -62 ±5° while the radial angular line 34 makes an angle from the vertical of about 36 ±5°. In general, the band length extends circumferentially around the posterior leaflet **PL** and around a portion of the anterior leaflet **AL,** namely a short distance past both the anterior commissure **AC** and the posterior commissure **PC.**

To help define this span, clock positions may be assigned relative to the major axis 22 and minor axis 24 of the mitral valve **MV;** that is, the vertical minor axis 24 extends between and defines 12:00 and 6:00, and the horizontal major axis 22 extends between and defines 3:00 and 9:00. Using this nomenclature, the band length illustrated in Figure 1 extends between radial line 32 at about 10:15 and radial line 34 at about 1:00. Of course, these geometries may be expressed in percent of a continuous ring or in degrees, and the aforementioned span 30 therefore is about 77% around the mitral annulus or about 278°.

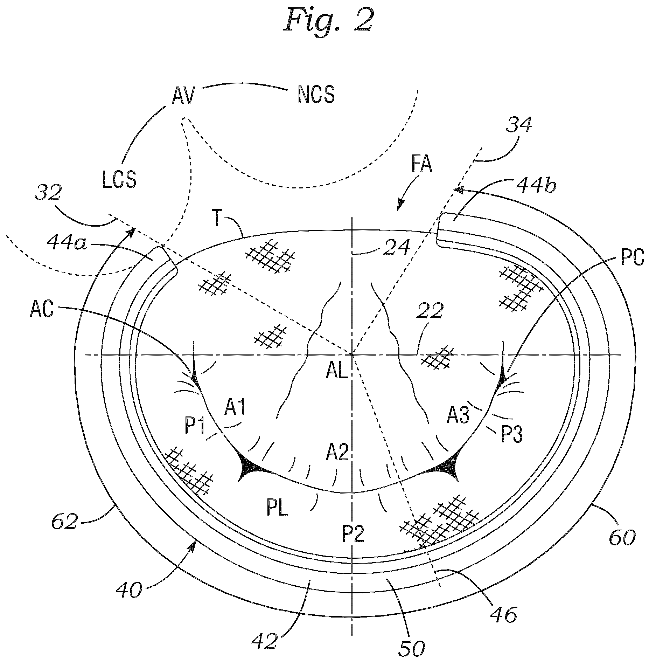

Figure 2 illustrates the mitral valve and anatomical landmarks with an exemplary annuloplasty band 40 secured thereto. The band 40 is preferably asymmetric when implanted in that it extends farther around one side of the mitral annulus than around the other. Mitral bands are typically asymmetric relative to the major axis 22 of the annulus, but the band 40 is also asymmetric relative to the minor axis 24. Looking down on the mitral valve as in Figure 2, the vertical minor axis 24 extends through the midpoint of both leaflets **AL, PL.** The annuloplasty band 40 is discontinuous with a mid-section 42 and two free ends 44a, 44b, one on either side of the minor axis 24. The band 40 asymmetrically extends farther CCW around the mitral annulus past the posterior commissure **PC** than CW past the anterior commissure **AC** so that its circumferential length to the right is larger or longer than to the left. Stated another way, the asymmetric position of the implanted band 40 is rotated in a counter-clockwise (CCW) direction around the mitral annulus from a symmetric position so that the circumferential center of the band (located at about number 46) lies CCW from the minor axis 24.

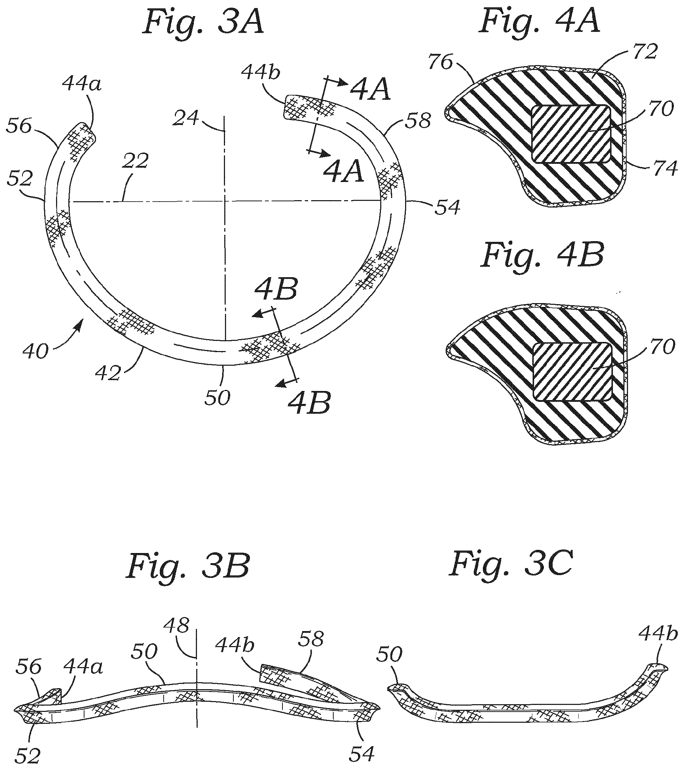

As seen in Figures 3A-3C, the exemplary annuloplasty band 40 has a gentle upward rise or bow 50 along a vertical axis 48 in its mid-section 42 that remains centered on the minor axis 24. (The vertical axis 48 is perpendicular to both the major axis 22 and minor axis 24 and extends through their intersection.) The bow 50 diminishes on either side of the minor axis 24 to low points 52, 54 around the ring along the major axis 22. The first free end 44a lies at the terminus of a first rising segment 56 on the anterior side of the band 40 extending up from the first low point 52. The second low point 54 occurs in the band mid-section 42 along the major axis 22 opposite to the first free low point 52. The band 40 then rises up in segment 58 from the second low point 52 toward the second free end 44b. If the band 40 were continuous without a gap between the free ends 44a, 44b, it would define a saddle shape with both the anterior and posterior sections bowed upward (convex up) with the sides that cross the major axis 22 being curved downward (convex down). Of course, this discussion refers to a relative orientation in which "up" corresponds to the left atrium and "down" to the left ventricle, so that blood flows downward through the annulus.

As seen in Figure 2 and the top view of Figure 3A, the upward bow 50 of the annuloplasty band 40 is centered on the minor axis 24 such that lengths of the band on either side of the high point of the bow are dissimilar. Specifically, as indicated in Figure 2, a first span 60 that extends counter-clockwise (CCW) from the high point of the bow 50 at the minor axis 24 is longer than a second span 62 that extends clockwise (CW). Specifically, the first span 60 extends past the posterior commissure **PC** of the mitral annulus and the second span 62 extends past the anterior commissure **AC.** Using the aforementioned expressions, the first span 60 extends CCW from the minor axis 24 to a farthest extent of about 1:00 or about 42% (about 150°) around the mitral annulus, while the second span 62 extends CW from the minor axis 24 to a farthest extent of about 10:15 or about 35% (about 128°) around the mitral annulus.

Figures 4A and 4B are sections views of the annuloplasty band 40 taken along corresponding section lines in Figure 3A. In a preferred example, the band construction includes a relatively rigid or semi-rigid inner core 70 surrounded by a suture-permeable interface. The interface may include an elastomeric sleeve 72 closely surrounding the core and a fabric outer cover 74, for example, a polyethylene terephthalate (PET) fabric cover. In the preferred example, the elastomeric sleeve 72, which may be silicone rubber, is molded to have a radially outwardly-extending flange 76 to facilitate suturing of the band 40 to the mitral annulus. The band 40 may be secured with sutures, staples, or other such devices to an inside ledge of the mitral annulus. In a typical procedure, an array of sutures is anchored through the annulus and then threaded through corresponding locations around the interface on the outside of the band 40, and then the band is parachuted down the suture array to be seated at the annulus before tying off the sutures.

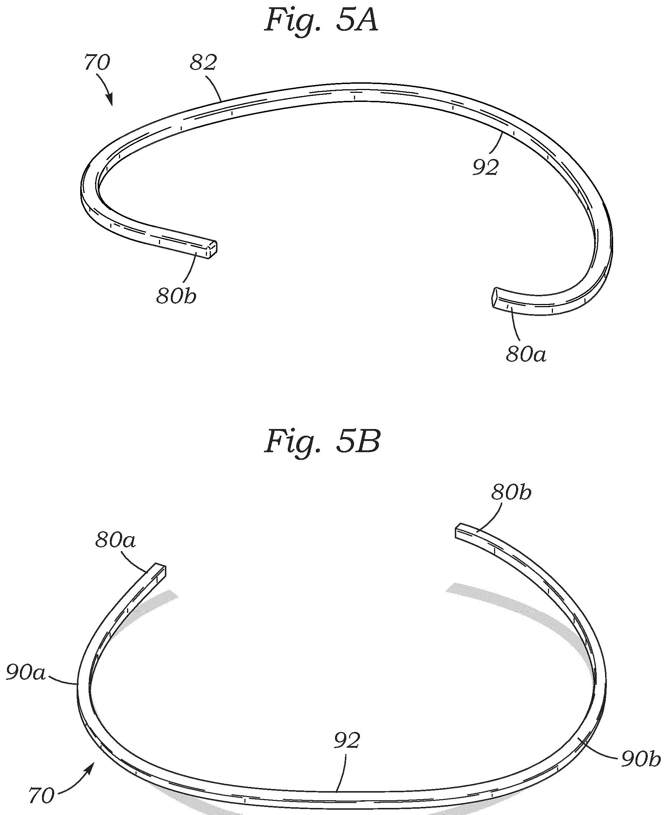

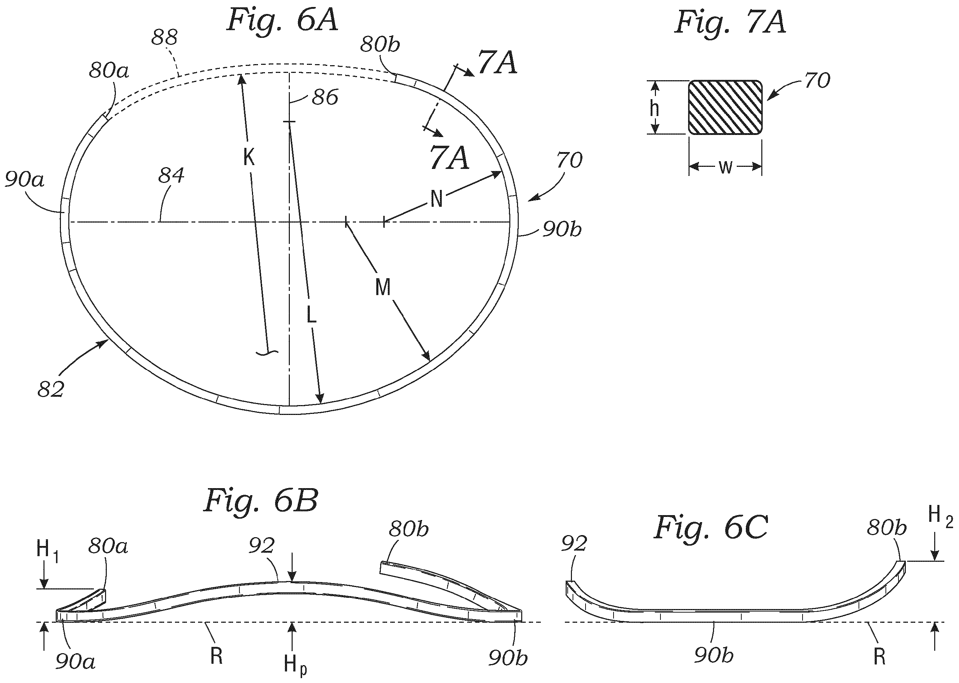

Figures 5A-5B and 6A-6C show an exemplary inner core 70 for the annuloplasty band 40. The shape of the core 70 defines the shape of the band 40 in general, as the outer suture-permeable interface simply conforms to the core. Accordingly, the core 70 is open or discontinuous with two free ends 80a, 80b at terminal ends of a mid-section 82. As seen in the plan view of Figure 6A, the peripheral shape of the core 70 is roughly oval or somewhat D-shaped, defining a major axis 84 and a minor axis 86. As oriented in Figure 6A, the core 70 has a continuous posterior portion below the major axis 84 and two abbreviated anterior segments above the major axis 84. If the anterior segments continued past the two free ends 80a, 80b they would meet, with the imaginary extension 88 being somewhat flatter or straighter than the posterior portion. The plan view shape of the core 70 is symmetric across the minor axis 86 as far as the first end 88a, though the second end 88b extends farther.

The plan view shape of the core 70 has a gradually changing curvature as indicated by the different radii of curvature drawn at discreet points. The posterior portion has a relatively large radius L which gradually diminishes as the core 70 continues around the periphery in both directions toward the anterior side from radius M to radius N. At this stage, the anterior segments straighten out so that the imaginary curvature 88 or the extension of the free ends 80a, 80b has a radius K which is larger than radius L. In other words, the core 70 has a rounded, convex contour along its entire span, with a more rounded posterior portion than an anterior portion.

Figure 6B illustrates the change in height of the ring relative to a reference plane R, which is formed by the intersection of the major axis 84 and minor axis 86. The core 70 has two low points 90a, 90b centered along the major axis 84, or at two opposite lateral sides. The two low points 90a, 90b lie in the reference plane R and the core 70 extends in parallel with the reference plane R for a short distance on both sides of the major axis 84, as seen at 90b in Figure 6C. That is, the core 70 has two planar segments on both lateral sides extending across the major axis 84.

The posterior portion has a smooth upward bow 92 that reaches a height Hₚ. The first free end 80a rises up to a height H₁, while the second free end 80b rises up to a height H₂. As mentioned, the plan view peripheral shape is symmetric across the minor axis 86 as far as the first end 88a. Likewise, the core 70 is symmetric in elevation across the minor axis 86 as far as the first end 88a. This is seen in Figure 6C where the first free end 80a is completely hidden behind the segment leading to the second free end 80b.

The core 70 may comprises a particular material with a constant cross-section along its length, and is shown rectangular in the illustrated example. As indicated by the sections shown in Figures 4A and 4B, and also in Figure 7, taken through different locations of the band 40 and core 70, the core is desirably the same size and shape in the mid-section 92 as it is throughout and to the free ends 80a, 80b thereof.

To provide some flexibility near the free ends 80a, 80b of the core 70 (and band 40) and help avoid dehiscence, or suture pull-out, the core 70 is formed of nitinol, a nickel-titanium alloy with particularly beneficial properties. First of all, examples of the annuloplasty bands 40 of the present disclosure are "generally rigid" in that the core 70 will resist distortion in the plane of the annulus when subjected to the stress imparted thereon by the mitral valve annulus of an operating human heart. In this sense, "distortion" means substantial permanent deformation from a predetermined or manufactured shape. However, at the same time, the core 70 is formed to have substantial flexibility out of the plane of the annulus to permit greater up-and-down motion. Stated another way, the core 70 is stiffer in the plane of the annulus than it is out of the plane of the annulus. In particular, the core 70 is formed so that the free ends will flex somewhat after implant to "ride out" the regular motion created by the systolic-diastolic contractions and expansions of the heart muscle.

To realize this unique combination of flexibility, the rings must provide enough stiffness to reshape the annulus that is adjacent to thick muscular structures. Although making these rings stiff in the plane of the annulus is necessary for them to fulfill their function, stiffening these rings in the out of plane axis increases the risk the sutures used to attach the ring will pull through the tissue or break as the native annulus shape changes throughout the cardiac cycle. For this reason, it is desirable to maximize the flexibility in the out of plane axis to reduce the risk of suture dehiscence or breakage while maintaining an optimum stiffness to remodel the annulus in the plane of the annulus. However, attempts to maximize the flexibility of the annuloplasty ring in the out of plane direction to reduce the stress on sutures have been limited by the need for adequate stiffness and fatigue resistance to permanently remodel the annulus.

In a preferred configuration to attain a differentiation in area moment of inertia, the core 70 is formed of nitinol where the area moment of inertia in the out of plane direction is much less than the area moment of inertia in the plane of the annulus. This makes the ring stiff enough to hold the annulus in the correct shape while being flexible enough out of plane to minimize the risk of suture dehiscence or breakage.

More specifically, the exemplary annuloplasty ring core 70 of the present application should have sufficient in-plane stiffness to "remodel" the mitral annulus; or, in other words, to withstand the cyclic forces applied to the annuloplasty ring during systole/diastole. A number of prior art annuloplasty rings have been proven to provide such remodeling stiffness. In particular, the Physio II^{®} annuloplasty ring available from Edwards Lifesciences, Corp. of Irvine, CA is the best-selling ring around the world for the mitral annulus. Consequently, the present application contemplates, in some implementations, a ring core 72 having in-plane stiffness substantially equivalent to the Physio II annuloplasty ring.

To assess the in-plane stiffness, both the Physio II^{®} annuloplasty ring and the annuloplasty band 40 were tested. Rigidity in both the anterior-to-posterior and commissure-to-commissure dimensions was assessed via compression testing. Each test object was placed upright and perpendicular to the tester platform and oriented in the proper direction. Each ring was stabilized while a force of up to 800 grams was applied. The displacement across the respective access was measured, and a stiffness value obtained by dividing the force applied by the resulting displacement. The following results, in metric units, were obtained for the in-plane stiffness of both the Physio II^{®} annuloplasty ring and the annuloplasty band 40.

**TABLE I: IN-PLANE STIFFNESS COMPARISON**

| *Band size (mm)* | *Physio II*^{®} *ring stiffness (N*/*mm)* | *Band 40 stiffness (N*/*mm)* |
|---|---|---|
| 24 | 0.78 | 0.90 |
| 26 | 0.48 | 0.80 |
| 28 | 0.72 | 0.70 |
| 30 | 0.71 | 0.60 |
| 32 | 0.81 | 0.60 |
| 34 | 0.90 | 0.60 |
| 36 | 0.90 | 0.60 |
| 38 | 0.72 | 0.60 |
| 40 | 0.85 | 0.60 |

Although the stiffness for the present band 40 is somewhat less for the larger rings than the Physio II^{®} ring stiffness the difference is not believed to be sufficient to eliminate the remodeling capacities of the ring. It is believed that an in-plane stiffness of between about 0.5-1 N/mm for any particular size of ring is believed to be sufficient for remodeling purposes, while also avoiding an excessively stiff ring without any flexibility whatsoever. Preferably, therefore, the in-plane stiffness should be greater than about 0.5 N/mm, and more preferably greater than or equal to about 0.6 N/mm and less than about 1 N/mm for most sizes. In some configurations the in-plane stiffness should be at least about 0.6 N/mm, or at least about 0.7 N/mm, or at least about 0.8 N/mm, or at least about 0.9 N/mm. Indeed, although the present band 40 has sufficient in-plane stiffness to remodel the mitral annulus, as explained below the out-of-plane stiffness is significantly less which enables the free ends two flex somewhat during the systole/diastole cycle. As explained elsewhere, this helps prevent dehiscence or suture pull out.

To quantify the out-of-plane stiffness, the present band 40 was compared with another commercial annuloplasty ring having an open or C-shaped configuration. This is because the out-of-plane stiffness is measured by applying opposed forces on the free ends of the open rings while holding the opposite closed side stationary. The particular commercial annuloplasty ring chosen for comparison is the CG Future^{®} annuloplasty ring from Medtronic, Corp. of Minneapolis, MN. The CG Future^{®} ring is an open ring, though it has an outer fabric cover which extends between the free ends. The outer fabric cover does not affect the out-of-plane stiffness.

Tests were thus conducted on two sizes of the present band 40 as well as the CG Future^{®} annuloplasty ring. The 28 mm ring size of the present band 40 had a measured out-of-plane stiffness of 0.027 N/mm, while the 28 mm CG Future^{®} ring had a measured out-of-plane stiffness of 0.062 N/mm. The 32 mm ring size of the present band 40 had a measured out-of-plane stiffness of 0.023 N/mm, while the 32 mm CG Future^{®} ring had a measured out-of-plane stiffness of 0.048 N/mm. This demonstrates that the present band 40 is significantly more flexible in out-of-plane bending then the CG Future^{®} ring.

Further tests were conducted on all sizes of the present band 40, with the results shown below:

**TABLE II: OUT-OF-PLANE STIFFNESS OF EXEMPLARY BAND 40**

| *Band size (mm)* | *Band 40 stiffness (N*/*mm)* |
|---|---|
| 24 | 0.049 |
| 26 | 0.041 |
| 28 | 0.027 |
| 30 | 0.028 |
| 32 | 0.023 |
| 34 | 0.022 |
| 36 | 0.014 |
| 38 | 0.015 |
| 40 | 0.012 |

It is believed that an out-of-plane stiffness of between about 0.01-0.05 N/mm is desirable to provide sufficient flexibility to the annuloplasty ring and avoid suture pull out. Preferably, therefore, the out-of-plane stiffness should be less than about 0.05 N/mm, more preferably less than about 0.04 N/mm for most sizes. It should be noted that the out-of-plane stiffness of the smaller sizes of the band 40 are proportionally larger than the stiffness of the larger bands, which is mainly a function of a larger moment arm between the free ends. That is, the test involves applying opposite axial forces to free ends, and the same force applied to both a small and a large band will result in a greater bending moment within the core of the band for the larger size.

One way to quantify the preferred differential stiffness of the core 70 is in the ratio between the out-of-plane stiffness versus the in-plane stiffness. The following table provides the ratios for the core 70 as tested:

**TABLE III: RATIO OF OUT OF PLANE STIFFNESS VERSUS IN PLANE STIFFNESS OF BAND 40**

| *Band size (mm)* | *Out-of-plane stiffness (N*/*mm)* | *In-plane stiffness (N*/*mm)* | *Ratio (%)* |
|---|---|---|---|
| 24 | 0.049 | 0.90 | 5.4 |
| 26 | 0.041 | 0.80 | 5.1 |
| 28 | 0.027 | 0.70 | 3.9 |
| 30 | 0.028 | 0.60 | 4.7 |
| 32 | 0.023 | 0.60 | 3.8 |
| 34 | 0.022 | 0.60 | 3.7 |
| 36 | 0.014 | 0.60 | 2.3 |
| 38 | 0.015 | 0.60 | 2.5 |
| 40 | 0.012 | 0.60 | 2.0 |

Accordingly, the bands 40 of the present application desirably have an out-of-plane stiffness to in-plane stiffness ratio of between about 2 and about 6%. In a preferred example, the bands 40 have an out-of-plane stiffness to in-plane stiffness ratio of greater than about 2%, and more preferably greater than about 3%, but no more than about 6%. In other examples, the bands 40 can have an out-of-plane stiffness to in-plane stiffness ratio of about 1%, about 1.5%, about 2%, about 2.5%, about 3%, about 3.5%, about 4%, about 4.5%, about 5%, about 5.5%, about 6%, about 6.5%, or about 7%. In yet further examples, the bands 40 can have an out-of-plane stiffness to in-plane stiffness ratio of between about 1% and about 7%, between about 2% and about 5.5%, between about 3.5% and about 5.5%, between about 2% and about 5%, between about 2.5% and about 5%, between about 3% and about 6%, between about 3% and about 5.5%, between about 2.5% and about 5.5%, between about 4% and about 6%, between about 3.5% and about 5%, or between about 2.5% and about 5.5%.

Nitinol is a preferred material for the core 70 as it has a modulus of elasticity or Young's modulus equal to the longitudinal stress divided by the strain that is much less than other metals used for annuloplasty bands. For instance, a preferred nitinol used for the core has a Young's modulus of about 70 GPa, while the Young's modulus for a typical titanium core would be about 113.8 GPa. Using a combination of greater radial to axial dimension cross-section and a highly flexible nitinol to stiffen the core in-plane but provide flexibility out of plane, the desired differentiation in area moment of inertia results.

Annuloplasty rings are commonly sized in even 2 mm increments between about 24 and 40 mm. Preferably, the present core 70 is made a little stiffer in-plane than prior rings in the smaller sizes because these smaller sizes are often used in ischemic cases where a stiffer ring is desired to resist higher annular dilatation forces. Conversely, in the larger sizes, the stiffness for the core 70 is made a little less than prior rings to reduce the profile of the ring which otherwise would have seemed too big from the perception of surgeons. As will be detailed below, therefore, the particular cross-sectional dimensions or ratios preferably vary for different sized rings.

The preferred geometry to accomplish this effect is a rectangular core 70 that is thicker in the radial direction than it is perpendicular to the annulus. This effect could be accomplished by many different shapes including a trapezoid, triangle, U-channel with the "U" opening upward or a U-channel with the "U" opening radially outward, though U-shaped cores can be somewhat more expensive to manufacture.

A rectangular shape can easily be machined with traditional end mills, laser cutting or abrasive water jet cutting. One preferred method of making these rings is to use drawn wire, rolled to the desired height to width ratio then heat set into the desired ring shape. Other alloys could be made from drawn and wrought wire, but the forging process may introduce defects including areas of residual tensile stress. Nitinol, in contrast, can be shaped and heat set in a mold with minimal risk of introducing defects or residual stress that could affect the fatigue life of the core.

In certain aspects, the method of making rings of the present disclosure from wire having the desired rectangular cross-section can provide technical benefits. Cutting a core 70 from a flat-sheet can introduce material defects/effects at or near the cutting surfaces due to the cutting process, such as heat-affected-zone changes to material that can be introduced by laser-cutting processes that cut by melting the nitinol. In contrast, a core 70 cut from a continuous wire having the desired rectangular cross-section may only have material defects/effects at or near the two cutting surfaces at the free ends of the wire. Any such defects at or near the free ends of the ring can be less problematic in terms of the fatigue life under cyclic loading anticipated by the annuloplasty ring after implantation. Avoiding the formation of material defects/effects along the entirety or substantially the entirety of the length of the core 70 can be advantageous in certain implementations.

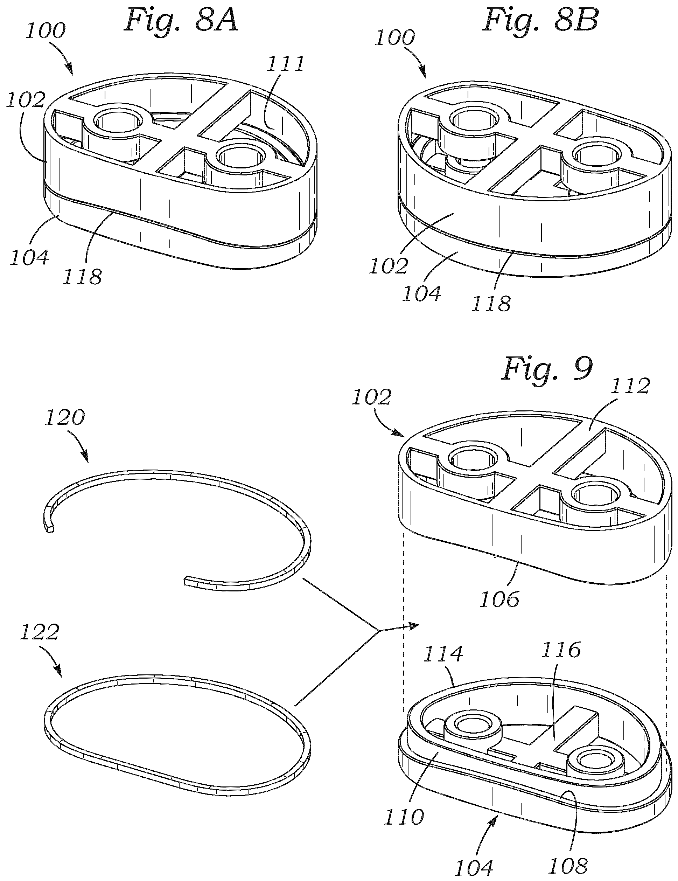

Figures 8A/8B and 9 illustrate a first example of a fixture 100 for forming the core 70. The fixture 100 includes a first or top half 102 and a second or bottom half 104. The top half 102 has a lower surface with a contoured outer ledge 106, and the bottom half 104 has an upper surface with a contoured outer ledge 108 (Figure 9). The top half 102 defines a lower cavity 111 that fits closely around an upper side wall 110 of the bottom half 104. The cavity 111 in the top half 102 ends at an inner frame 112 which is positioned to contact a top edge 114 of the bottom half 104 when the contoured outer ledge 106 is spaced a small distance above the contoured outer ledge 108 of the bottom half 104 to form a consistent contoured gap 118 (Figures 8A/8B). The bottom half 104 also has an inner frame 116. The two halves 102, 104 may be secured together using fasteners between the inner frames 112, 116, such as screws through the holes shown.

To form a core 70 using the fixture 100, the two halves 102, 104 are separated and a blank or billet placed around the contoured outer ledge 108 of the bottom half 104. A discontinuous or C-shaped blank 120 may be used, having previously been cut to size. Alternatively, a continuous or closed blank 122 may be used. If the latter, the final core 70 requires a second step of cutting and polishing the blank 122 after removal from the fixture 100. The blanks 120, 122 are prepared from nitinol sheet. That is, a plan view of the core 70 is used to cut out the blanks 120, 122 from a flat sheet, resulting in a rectangular cross-section. The blanks 120, 122 may be polished such as by electropolishing at this stage, or after the core 70 is formed as explained below.

After placing the blank 120 or 122 around the contoured outer ledge 108 of the bottom half 104, the technician lowers the top half 102 onto the bottom half until the contoured outer ledge 106 contacts the blank 120 or 122. Because the blanks 120, 122 are flat and the contoured outer ledges 106, 108 are undulating, the top half 102 must be forced down to bend the respective blank 120 or 122 to conform to the consistent contoured gap 118. This may be done with a weight or by screwing the two inner frames 112, 116 together with screws. Ultimately, the inner frame 112 contacts the top edge 114 and the matching outer ledges 106, 108 define the consistent contoured gap 118 which has a height equal to the height of the respective blank 120 or 122. This forces the blank 120 or 122 into the desired shape.

Subsequently, a heat-treating step is performed to shape set the blank 120 or 122 into the shape of the core 70. The particular temperatures and durations of the heat treatment may vary depending on the specific nitinol used and the cross-sectional dimensions of the respective blank 120 or 122. However, the heat treatment is sufficient to change the crystalline structure of the blank 120 or 122 so that it retains the contoured shape. The top half 102 is then removed from the bottom half 104 and the shaped blank 120 or 122 can be removed. If a continuous blank 122 is used it must be trimmed down to size. The fixture 100 is desirably made of aluminum, steel or another suitable metal that will withstand repeated heat treatments. The blank 120 or 122 is polished such as by electropolishing once formed to remove any sharp edges.

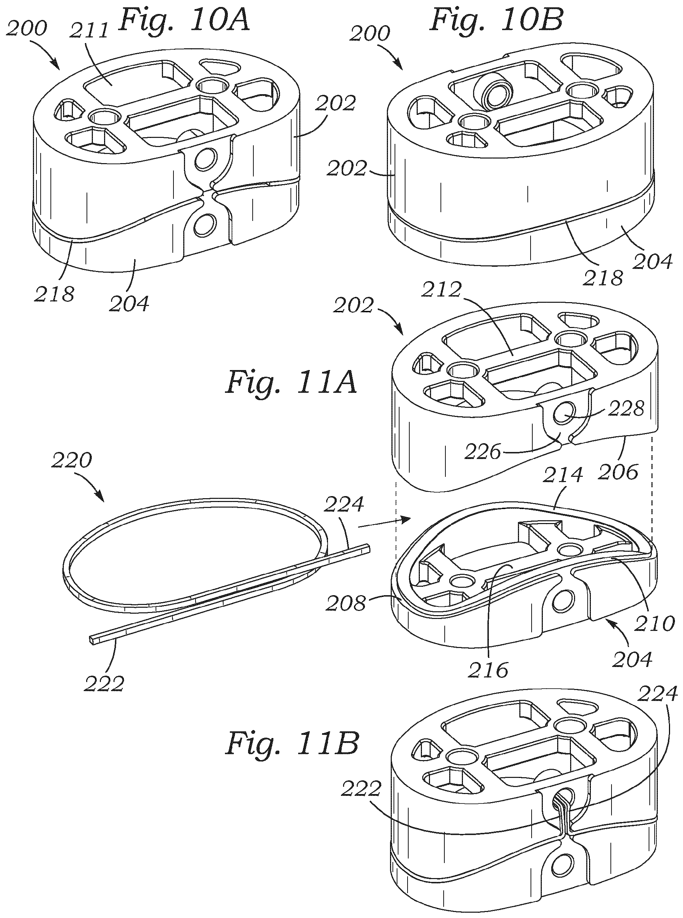

A second type of fixture 200 seen in Figure 10A and 10B may be used to make cores 70 with nitinol wire. The fixture 200 again includes a first or top half 202 and a second or bottom half 204. The top half 202 has a lower surface with a contoured outer ledge 206, and the bottom half 204 has an upper surface with a contoured outer ledge 208 (Figure 11A). The top half 202 defines a lower cavity 211 below an inner frame 212 that fits closely around an upper step 210 of the bottom half 204. The cavity 211 in the top half 202 may have an inset horizontal edge (not shown) that is positioned to contact a top edge 214 of the bottom half 204 when the contoured outer ledge 206 is spaced a small distance above the contoured outer ledge 208 of the bottom half 204 to form a consistent contoured gap 218 (Figures 10A/10B). The bottom half 204 also has an inner frame 216. The two halves 202, 204 may be secured together using fasteners between the inner frames 212, 216, such as screws through the holes shown.

A rectangular wire blank 220 may be clamped within the fixture 200 to from the core 70. The wire blank 220 is oversized so as to have two overlapping free ends 222, 224. The wire blank 220 is initially planar and is wrapped around the upper step 210 above the contoured outer ledge 208 of the bottom half 204. The top half 202 is the lowered onto the bottom half 204. The free ends 222, 224 are fed upward from the gap 218 formed between the contoured outer ledges 206, 208 along a recess 226 (Figure 11A) in a side wall of the bottom half 204 and inserted into a through hole 228 leading into the cavity 211 in the top half 202 (Figure 11B). The top half 202 is then forced down to bend the wire blank 220 to conform to the consistent contoured gap 218. This may be done with a weight or by screwing the two inner frames 212, 216 together with screws. Although not shown, the free ends 222, 224 of the wire 220 are then secured within the fixture 200 to ensure the wire is taut.

At this stage, the aforementioned heat-treating step is performed to shape set the wire blank 220 into the 3D shape of the core 70. The formed blank 220 must be finally trimmed down to size and polished such as by electropolishing once formed to remove any sharp edges.

Examples of the annuloplasty bands of the present disclosure may be configured to correct particular pathologies or adjust for different annulus sizes. That is, some examples provide a set of bands defined by band bodies wherein the proportional shapes of the band bodies may change with increasing nominal orifice sizes of the band bodies in the set. The orifice size generally refers to the nominal length across the major axis of the band body, although some rings or bands deviate from this nomenclature. Typically, annuloplasty rings and bands have orifice sizes in even millimeter increments (*e.g.,* 24 mm, 26 mm, *etc.,* up to about 40 mm) as measured across the major axes. Other sizing schemes are also possible, for example, odd millimeter increments, every millimeter increments, or combination schemes, for example, every millimeter up to a certain size, then even increments above that size. Such rings will have distinct packaging so as to be labeled with the particular size.

The change of band shape depends on the pathology or annulus size being corrected. For instance, pathologies resulting in mitral regurgitation may benefit from a set of bands which have increasing circularity as the band size increases. It is important to understand that the set of bands is formed of band bodies that are formed during manufacture to be "generally rigid" and not easily manipulated. It should also be mentioned that holders for such annuloplasty bands have peripheral shapes that conform to the optimally-sized bands.

For instance, an exemplary set of cores 70 for the bands 40 described herein may have the following dimensions:

**TABLE IV: EXEMPLARY CORE PLAN VIEW DIMENSIONS**

| *Band size (mm)* | *Major axis length (in.)* | *Minor axis length (in.)* | *Minor:major ratio (%)* | *Free end gap (in.)* | *Gap:major ratio (%)* |
|---|---|---|---|---|---|
| 24 | 0.946 | 0.643 | 68.0 | 0.584 | 61.7 |
| 26 | 1.024 | 0.688 | 67.2 | 0.635 | 62.0 |
| 28 | 1.102 | 0.733 | 66.5 | 0.686 | 62.2 |
| 30 | 1.182 | 0.790 | 66.8 | 0.735 | 62.2 |
| 32 | 1.260 | 0.848 | 67.3 | 0.784 | 62.2 |
| 34 | 1.340 | 0.909 | 67.8 | 0.830 | 61.9 |
| 36 | 1.418 | 0.984 | 69.4 | 0.885 | 62.4 |
| 38 | 1.496 | 1.045 | 69.9 | 0.944 | 63.1 |
| 40 | 1.576 | 1.126 | 71.4 | 0.980 | 62.2 |

For this table, the major axis length is measured across the major axis 84 (Figure 6A) of the core 70, between the inside surfaces of the core. That is, the major axis length roughly corresponds to the orifice size across the major axis, ignoring the suture-permeable interface around the core, which is also about the same as the nominal or labeled ring size. For an example of a size 24 ring the major axis length is 0.946 in, or 24.03 mm. The minor axis length, on the other hand, is not measured across the minor axis 86 since there is no portion of the core 70 on the anterior side that intersects the minor axis. Instead, the minor axis length is measured between the inside of the posterior mid-section 82 at the minor axis 86 to a projection of the extent of the longer second free end 80b to the minor axis 86. Likewise, the gap between the free ends 80a, 80b is that distance parallel to the major axis 84 between the free ends 80a, 80b.

The plan view dimensions illustrate some deviation of the ratio between the minor axis length and the major axis length across ring sizes, though not a significant amount. The ratio decreases slightly from the smallest band, 24 mm, to a minimum at 28 mm, then gradually increases to the largest band size of 40 mm. Throughout the size range, the ratio of the gap size to the major axis length remains nearly constant, within a range of about 61-63%.

In another example, a set of bands has increasing saddle profiles for larger sizes, though not linearly increasing. That is, a preferred set of bands has a relatively flat saddle (smaller upward bows) for bands under about 30 mm, a constant moderate saddle shape in bands of from about 24-30 mm, while the larger bands from about 36-40 mm have more pronounced saddles. The following table is illustrative:

**TABLE V: EXEMPLARY CORE ELEVATIONAL DIMENSIONS**

| *Band Size (mm)* | *Posterior bow height, H_{P} (in.)* | *First free end height, H₁ (in.)* | *1st free end:post. bow ratio (%)* | *2nd free end height, H₂ (in.)* | *2nd free end:post. bow ratio (%)* |
|---|---|---|---|---|---|
| 24 | 0.052 | 0.037 | 71.2 | 0.064 | 123.1 |
| 26 | 0.063 | 0.039 | 61.9 | 0.070 | 111.1 |
| 28 | 0.073 | 0.051 | 70.0 | 0.084 | 115.1 |
| 30 | 0.084 | 0.059 | 70.2 | 0.101 | 120.2 |
| 32 | 0.095 | 0.069 | 72.6 | 0.129 | 135.8 |
| 34 | 0.106 | 0.081 | 76.4 | 0.172 | 162.3 |
| 36 | 0.119 | 0.094 | 79.0 | 0.221 | 185.7 |
| 38 | 0.121 | 0.098 | 81.0 | 0.226 | 186.8 |
| 40 | 0.130 | 0.098 | 75.4 | 0.255 | 196.2 |

Table V shows the absolute increases in heights around the core 70, but also some variation in the ratio between the heights H₁, H₂ of the free ends 80a, 80b and the posterior side rise H_{P} (see Figure 6B). Namely, the ratio between the height H₁, H₂ of both free ends and the posterior side rise H_{P} initially decreases from the smallest band, 24 mm, to a minimum at 26 mm, then generally increases to the largest band size of 40 mm. The ratio between the height H₁ of the first free end 80a increases more slowly to the band size of 38 mm, then drops down a bit in the largest band size of 40 mm. On the other hand, the ratio between the height H₂ of the second free end 80b continually increases from 26 mm to the largest band size of 40 mm. The flatter cores 70 used for the smaller annulus sizes reflects an understanding of the average anatomy, as does the more pronounced rises for the free ends in the larger sizes. In other words, the saddle shape varies with ring size. The smaller sizes have flatter saddles and the larger sizes have more pronounced saddles.

Finally, the cross-sectional thickness of the core 70 itself also may vary for different band sizes. That is, the core 70 is preferably a little stiffer in-plane than conventional annuloplasty bands/rings in the smaller sizes, because these smaller sizes are often used in ischemic cases where a stiffer ring is desired to resist higher annular dilatation forces. Conversely, in the larger sizes, the in-plane stiffness for the core 70 is desirably a little less than conventional annuloplasty bands/rings to reduce the profile of the final band 40, which otherwise might seem too big in the perception of surgeons.

However, the in-plane stiffness does not simply depend on the cross-sectional dimensions, since the in-plane stiffness is a measure of how stiff is the core when a bending force in the plane of the core is applied. That stiffness partly depends on the size of the ring, as the relevant opposing forces are applied in the plane of the ring, or commissure-to-commissure, so as to create a bending moment in the ring. To determine a preferred cross-section, tests for stiffness of the core involved loading one end of the ring. Larger rings have longer lever arms and the cross-section needs to be adjusted so that the target ring stiffness is achieved. Therefore, the smaller ring s with shorter lever arms would have smaller cross-sections but higher stiffness (in-plane).

The following table is illustrative:

**TABLE VI: EXEMPLARY CORE CROSS-SECTIONAL DIMENSIONS**

| *Band size (mm)* | *Core radial width, w (in.)* | *Core axial height, h (in.)* | *Radial width:axial height ratio (%)* |
|---|---|---|---|
| 24 | 0.31 | 0.24 | 129 |
| 26 | 0.31 | 0.24 | 129 |
| 28 | 0.31 | 0.24 | 129 |
| 30 | 0.31 | 0.24 | 129 |
| 32 | 0.32 | 0.25 | 128 |
| 34 | 0.35 | 0.25 | 140 |
| 36 | 0.35 | 0.28 | 125 |
| 38 | 0.36 | 0.26 | 138 |
| 40 | 0.43 | 0.28 | 153 |

While the foregoing is a complete description of preferred examples, various alternatives, modifications, and equivalents may be used. Moreover, it will be obvious that certain other modifications may be practiced within the scope of the appended claims.

In the following the further embodiments are disclosed:
1. An annuloplasty band adapted for implant against a mitral valve annulus, the mitral valve annulus having a posterior aspect to which a posterior leaflet attaches and an anterior aspect to which an anterior leaflet attaches, the mitral valve annulus generally defining a D- or kidney-shape looking at an inflow side thereof with the anterior aspect being straighter than the more rounded posterior aspect, and a minor axis intersecting and extending across the mitral valve annulus between mid-points on the anterior and posterior aspects being shorter than a major axis perpendicular thereto intersecting and extending across the mitral valve annulus, wherein an intersection of the minor and major axes defines a reference plane, comprising:
   an inner core formed of nitinol and covered by a suture-permeable interface, the inner core having a shape such that when the ring is implanted around the mitral annulus the core has a continuous posterior mid-section that extends around on each side past the major axis in first and second segments terminating in first and second free ends, respectively, the first and second segments being of different length, the core having a constant cross-section throughout which is stiffer in bending within the reference plane than bending out of the reference plane.
2. An annuloplasty band adapted for implant against a mitral valve annulus, the mitral valve annulus having a posterior aspect to which a posterior leaflet attaches and an anterior aspect to which an anterior leaflet attaches, the mitral valve annulus generally defining a D- or kidney-shape looking at an inflow side thereof with the anterior aspect being straighter than the more rounded posterior aspect, and a minor axis intersecting and extending across the mitral valve annulus between mid-points on the anterior and posterior aspects being shorter than a major axis perpendicular thereto intersecting and extending across the mitral valve annulus, wherein an intersection of the minor and major axes defines a reference plane, comprising:
   an inner core formed of nitinol and covered by a suture-permeable interface, the inner core having a shape such that when the ring is implanted around the mitral annulus the core has a continuous posterior mid-section that extends around on each side past the major axis in first and second segments terminating in first and second free ends, respectively, the core having a constant rectangular cross-section throughout which is oriented to have a width generally parallel to the plane defined by the intersection of the major and minor axes, and a height which is perpendicular thereto, and the width is larger than the height.
3. An annuloplasty band adapted for implant against an atrioventricular valve annulus, comprising:
   a one piece inner core formed of nitinol and covered by a suture-permeable interface, the inner core having a shape such that when the ring is implanted around the valve annulus the core has a continuous posterior mid-section that extends around on each side and terminates in first and second free ends, wherein the core has a constant rectangular cross-section throughout which is oriented to have a width generally parallel to a plane which the ring primarily defines, and a height which is perpendicular thereto, and the width is larger than the height, wherein the inner core has an in-plane stiffness of at least about 0.5 N/mm.
4. The annuloplasty band of any of embodiments 1 to 3, wherein the inner core has an in-plane stiffness of between about 0.5 and about 1 N/mm.
5. The annuloplasty band of any of embodiments 1 to 3, wherein the inner core has an in-plane stiffness of at least about 0.6 N/mm.
6. The annuloplasty band of any of embodiments 1 to 3, wherein the inner core has an in-plane stiffness of at least about 0.7 N/mm.
7. The annuloplasty band of any of embodiments 1 to 3, wherein the inner core has an in-plane stiffness of at least about 0.8 N/mm.
8. The annuloplasty band of any of embodiments 1 to 7, wherein the inner core has an out-of-plane stiffness to in-plane stiffness ratio of greater than about 2.5%.
9. The annuloplasty band of any of embodiments 1 to 7, wherein the inner core has an out-of-plane stiffness to in-plane stiffness ratio of greater than about 3%.
10. The annuloplasty band of any of embodiments 1 to 7, wherein the inner core has an out-of-plane stiffness to in-plane stiffness ratio of greater than about 2%.
11. The annuloplasty band of any of embodiments 1 to 10, wherein the inner core has an out-of-plane stiffness to in-plane stiffness ratio of no more than about 6%.
12. The annuloplasty band of any of embodiments 1 to 7, wherein the inner core has an out-of-plane stiffness to in-plane stiffness ratio of about 1%.
13. The annuloplasty band of any of embodiments 1 to 7, wherein the inner core has an out-of-plane stiffness to in-plane stiffness ratio of about 1.5%.
14. The annuloplasty band of any of embodiments 1 to 7, wherein the inner core has an out-of-plane stiffness to in-plane stiffness ratio of about 2%.
15. The annuloplasty band of any of embodiments 1 to 7, wherein the inner core has an out-of-plane stiffness to in-plane stiffness ratio of about 2.5%.
16. The annuloplasty band of any of embodiments 1 to 7, wherein the inner core has an out-of-plane stiffness to in-plane stiffness ratio of about 3%.
17. The annuloplasty band of any of embodiments 1 to 7, wherein the inner core has an out-of-plane stiffness to in-plane stiffness ratio of about 3.5%.
18. The annuloplasty band of any of embodiments 1 to 7, wherein the inner core has an out-of-plane stiffness to in-plane stiffness ratio of about 4%.
19. The annuloplasty band of any of embodiments 1 to 7, wherein the inner core has an out-of-plane stiffness to in-plane stiffness ratio of about 4.5%.
20. The annuloplasty band of any of embodiments 1 to 7, wherein the inner core has an out-of-plane stiffness to in-plane stiffness ratio of about 5%.
21. The annuloplasty band of any of embodiments 1 to 7, wherein the inner core has an out-of-plane stiffness to in-plane stiffness ratio of about 5.5%.
22. The annuloplasty band of any of embodiments 1 to 7, wherein the inner core has an out-of-plane stiffness to in-plane stiffness ratio of about 6%.
23. The annuloplasty band of any of embodiments 1 to 7, wherein the inner core has an out-of-plane stiffness to in-plane stiffness ratio of about 6.5%.
24. The annuloplasty band of any of embodiments 1 to 7, wherein the inner core has an out-of-plane stiffness to in-plane stiffness ratio of about 7%.
25. The annuloplasty band of any of embodiments 1 to 7, wherein the inner core has an out-of-plane stiffness to in-plane stiffness ratio of between about 1% and about 7%
26. The annuloplasty band of any of embodiments 1 to 7, wherein the inner core has an out-of-plane stiffness to in-plane stiffness ratio of between about 2% and about 5.5%.
27. The annuloplasty band of any of embodiments 1 to 7, wherein the inner core has an out-of-plane stiffness to in-plane stiffness ratio of between about 3.5% and about 5.5%.
28. The annuloplasty band of any of embodiments 1 to 7, wherein the inner core has an out-of-plane stiffness to in-plane stiffness ratio of between about 2% and about 5%.
29. The annuloplasty band of any of embodiments 1 to 7, wherein the inner core has an out-of-plane stiffness to in-plane stiffness ratio of between about 2.5% and about 5%.
30. The annuloplasty band of any of embodiments 1 to 7, wherein the inner core has an out-of-plane stiffness to in-plane stiffness ratio of between about 3% and about 6%.
31. The annuloplasty band of any of embodiments 1 to 7, wherein the inner core has an out-of-plane stiffness to in-plane stiffness ratio of between about 3% and about 5.5%.
32. The annuloplasty band of any of embodiments 1 to 7, wherein the inner core has an out-of-plane stiffness to in-plane stiffness ratio of between about 2.5% and about 5.5%.
33. The annuloplasty band of any of embodiments 1 to 7, wherein the inner core has an out-of-plane stiffness to in-plane stiffness ratio of between about 4% and about 6%.
34. The annuloplasty band of any of embodiments 1 to 7, wherein the inner core has an out-of-plane stiffness to in-plane stiffness ratio of between about 3.5% and about 5%.
35. The annuloplasty band of any of embodiments 1 to 7, wherein the inner core has an out-of-plane stiffness to in-plane stiffness ratio of between about 2.5% and about 5.5%.
36. A method of forming the annuloplasty band as defined in any of embodiments 1-35, including:
   laser cutting a planar blank from a sheet of nitinol having an approximate peripheral length of the inner core,
   bending the planar blank to a 3D shape to form the core, and
   heat treating the core.
37. The method of embodiment 36, further including repeating the steps of bending and heat-treating multiple times to avoid placing undue strains/stresses on the blank during bending.
38. The method of any of embodiments 36 to 37, wherein the step of bending comprises clamping the blank between two fixtures having opposed surfaces that define the 3D shape.
39. The method of any of embodiments 36 to 38, wherein the step of heat-treating is done while the blank is clamped between the two fixtures.
40. A method of forming the annuloplasty band as defined in any of embodiments 1-35, including:
   cutting a rectangular-cross-sectioned wire to a length,
   bending the wire to a 3D shape to form the core, and
   heat treating the core.
41. The method of embodiment 40, further including repeating the steps of bending and heat-treating two or more times to avoid placing undue strains/stresses on the wire during bending steps between heat treating steps.
42. The method of any of embodiment 40 to 41, wherein the step of bending comprises clamping the wire between two fixtures having opposed surfaces that define the 3D shape.
43. The method of any of embodiments 40 to 42, wherein the step of heat-treating is done while the wire is clamped between the two fixtures.
44. The method of any of embodiments 40 to 43, wherein the wire is cut to a length having an approximate peripheral length of the inner core.
45. The method any of embodiments 40 to 44, wherein the wire is cut to a length longer than a peripheral length of the inner core, and free ends of the wire are inserted into and held within an inner cavity in one of the fixtures.

## Claims

1. A method of forming a C-shaped annuloplasty band (40) with a core (70) formed from Nitinol and having a constant cross-section with a wider radial dimension (w) than an axial dimension (h):
cutting a Nitinol wire (220) having the desired cross-section of the core (70) to a length;
bending the wire (220) to a 3D shape to form the core (70); and
heat treating the core (70).

2. The method of claim 1, wherein the constant cross-section is rectangular.

3. The method of claim 1, wherein the constant cross-section is a shape other than a rectangular shape, including a trapezoid, a triangle, or a U-shaped channel.

4. The method of any one of the preceding claims, further including repeating the steps of bending and heat-treating two or more times to avoid placing undue strains/stresses on the wire (220) during bending steps between heat treating steps.

5. The method of any one of the preceding claims, wherein the step of bending comprises clamping the wire (220) between two fixtures (202, 204) having opposed surfaces that define the 3D shape.

6. The method of any one of the preceding claims, wherein the step of heat-treating is done while the wire (220) is clamped between the two fixtures (202, 204).

7. The method of any one of the preceding claims, wherein the wire (220) is cut to a length having an approximate peripheral length of the inner core (70).

8. The method of any one of claims 1 to 6, wherein the wire (220) is cut to a length longer than a peripheral length of the inner core (70), and free ends (222, 224) of the wire (220) are inserted into and held within an inner cavity in one of the fixtures (202, 204).

9. The method of any one of the preceding claims, wherein the core (70) is oriented to have a width (w) generally parallel to a plane which the band (40) primarily defines, and a height (h) which is perpendicular thereto, and the core (70) has an in-plane stiffness of at least about 0.5 N/mm.

10. The method of claim 9, wherein the core (70) has an in-plane stiffness of between about 0.5 and about 1 N/mm, preferably an in-plane stiffness of at least about 0.6 N/mm, at least about 0.7 N/mm, or at least about 0.8 N/mm.

11. The method of any one of the preceding claims, wherein the core (70) has an out-of-plane stiffness to in-plane stiffness ratio of greater than about 2.5%.

12. The method of any one of the preceding claims, wherein the core (70) is covered by a suture-permeable interface (74).

13. The method of any one of the preceding claims, wherein the core (70) has a shape such that when the band (40) is implanted around the mitral annulus the core (70) has a continuous posterior mid-section (42) that extends around on each side past the major axis (22) in first and second segments (60, 62) terminating in first and second free ends (44a, 44b), respectively.

14. The method of claim 13, wherein the first and second segments (60, 62) are of different length.

15. An annuloplasty band (40) obtained by the method of any one of the preceding claims.
